# FASCICULE DE BREVET EUROPEEN

(11) **EP 0 589 741 B1**
(45) Date de publication et mention de la délivrance du brevet: **11.03.1998**
(21) Numéro de dépôt: 93402136.1
(22) Date de dépôt: 02.09.1993
(51) Int. Cl.: A61K 38/00, A61K 31/47

(54) **Utilisation d'un inhibiteur direct de la thrombine pour la fabrication d'un médicament à activité thrombolytique**
Verwendung eines direkten Inhibitors von Thrombin zur Herstellung eines Arzneimittels mit thrombolytischer Aktivität
Use of a direct inhibitor of thrombin for the production of a drug with thrombolytic activity

(30) Priorité: 11.09.1992 FR 9210833
(43) Date de publication de la demande: 30.03.1994
(73) Titulaire: SYNTHELABO, 92350 Le Plessis Robinson (FR)
(72) Inventeur: Berry, Christopher, F-94500 Champigny S/Marne (FR); Ferrari, Patrice, F-78390 Bois d'Arcy (FR)
(74) Mandataire: Thouret-Lemaitre, Elisabeth

(56) Documents cités:
- S. BUDAVARI ET AL. (EDS.) 'The Merck Index (11th edition)' 1989 , MERCK & CO., INC. , RAHWAY NJ, ETATS-UNIS * page 123, numéro 804 "Argatroban" *
- S. BUDAVARI ET AL. (EDS.) 'The Merck Index (11th edition)' 1989 , MERCK & CO., INC. , RAHWAY NJ, ETATS-UNIS * page 1220, numéro 7702 "PPACK" *
- BIOCHEMICAL AND BIOPHYSICAL RESEARCH COMMUNICATIONS vol. 179, no. 3 , 30 Septembre 1991 , ORLANDO FL, ETATS-UNIS pages 1500 - 1508 A. CHIU ET AL. 'Inhibition of the thrombin-platelet reactions by DuP 714.'

## Description

La présente invention a pour objet l'utilisation d'un inhibiteur direct de la thrombine pour la préparation d'un médicament à activité thrombolytique.

Les composés qui inhibent la thrombine par une interaction avec le site actif de cette protéase sont utilisés ou sont en cours de développement comme agents antithrombotiques. Des exemples de tels composés incluent l'argatroban (Okamoto S. et al, Biochem. Biophys. Res. Commun. **101**, 440, 1981. inter alia), le PPACK (Kettner C.A. et Shaw E.N., Thromb. Res. **14**, 969, 1979 inter alia), le DUP 714 (Kettner C.A., et al J. Biol. Chem. **265**, 18289, 1990 inter alia) et les hirudines (Rydel T.J. et al, Science **245**, 277, 1989 inter alia).

En cas d'infarctus du myocarde, de thrombose artérielle ou veineuse, ou d'embolie pulmonaire, les inhibiteurs de la thrombine peuvent accélérer la lyse et empêcher la réocclusion après traitement par un agent thrombolytique exogène tel que l'activateur de plasminogène recombinant (rTPA) ou la streptokinase.

Selon l'invention, la demanderesse a étudié l'activité des inhibiteurs directs de la thrombine, *per se,* comme agents thrombolytiques en l'absence d'activateurs de plasminogène exogenes.

Les composés utilisables selon l'invention ont été étudiés chez le rat quant à leur activité comme agents thrombolytiques dans un modèle de thrombose occlusive de l'aorte induite par une stimulation électrique.

On anesthésie au pentobarbital sodique (60 mg/kg i.p.) des rats CD de 450 g à 500 g et on cathètérise l'artère rénale gauche. On induit la thrombose selon la méthode décrite par Hladovec (Thromb. Haemosts., (1971), 26, 407-410). On libère un segment d'aorte abdominale d'environ 1 cm de longueur entre l'artère rénale et la bifurcation iliaque, on y place une sonde électromagnétique pour mesurer le débit sanguin et une électrode bipolaire en aval. Après 15 minutes de stabilisation, on applique à l'aide d'un stimulateur un courant direct de 5 mA, pendant 5 minutes, à la surface externe de l'artère. Lorsque le débit atteint la valeur zéro, préalablement déterminée par clampage de l'artère entre la sonde et l'électrode, on note le temps de formation de l'occlusion thrombotique. Après 5 minutes de stabilisation du thrombus, on injecte par le cathéter le composé à tester ou le véhicule sous un bolus de 0,5 ml suivi d'une perfusion de 60 minutes à 0,1 ml/min. On enregistre le débit sanguin pendant le temps de la perfusion. On estime la thrombolyse en mesurant l'aire sous la courbe qui dépend du temps nécessaire au début de la recanalisation et de l'importance de celle-ci. On exprime les résultats en moyenne ± s.e.m.

Chez les animaux recevant le véhicule, l'aorte abdominale reste occluse pendant toute la période de l'administration. En revanche, les inhibiteurs directs de la thrombine augmentent de manière dose-dépendante l'aire sous la courbe et restaurent rapidement le débit sanguin.

La demanderesse a plus particulièrement testé les substances suivantes : l'argatroban, la D-Phe-Pro-Arg-chlorométhylcétone (PPACK) et le Ac-(D-Phe)-Pro-boro-Arg-OH (DUP 714) de formule (1) 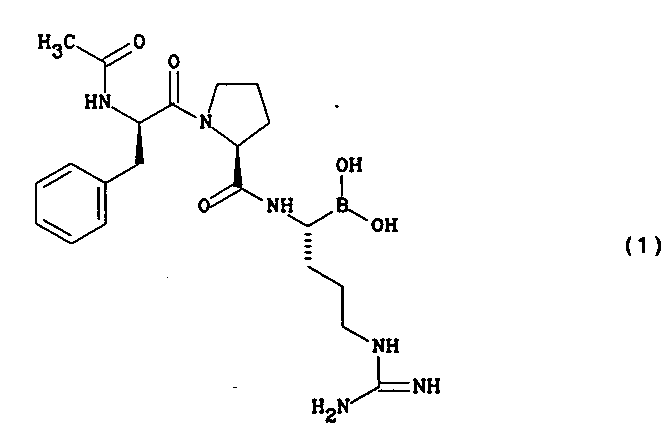 qui ont montré une activité thrombolytique.

Les résultats sont donnés dans le tableau de la page 4.

Les résultats des essais montrent que les inhibiteurs directs de la thrombine peuvent être utilisés pour la fabrication de médicaments à activité thrombolytique.

Ils peuvent être utilisés dans les pathologies qui impliquent l'utilisation d'un agent thrombolytique telles que l'infarctus du myocarde, les artérites des membres inférieurs, la thrombose veineuse profonde et l'embolie pulmonaire.

A cet effet ils peuvent être présentés sous toutes formes pharmaceutiques adaptées à l'administration intraveineuse, en association avec des excipients appropriés, dosées pour permettre une administration journalière de 10 à 500 mg de substance active.

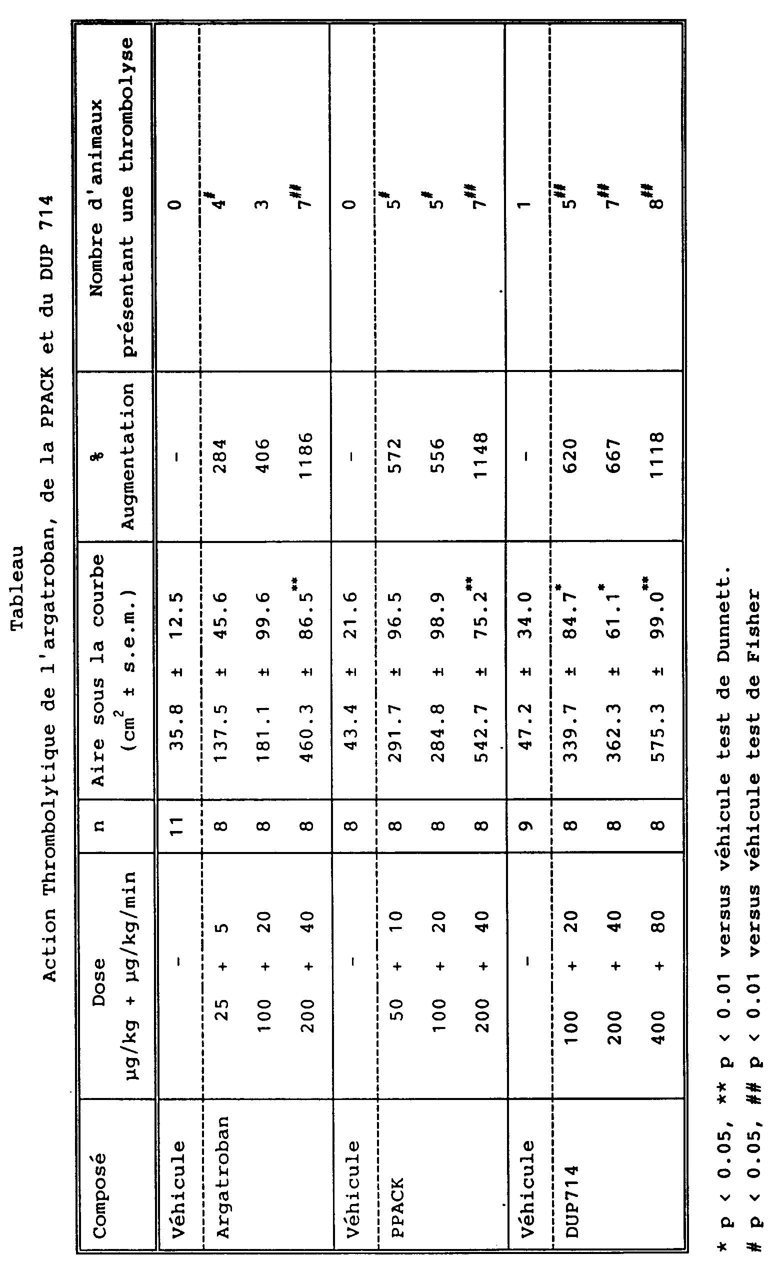

## Revendications

1. Utilisation d'un inhibiteur agissant au site actif de la thrombine pour la fabrication d'un médicament à activité thrombolytique, en l'absence d'activateurs de plasminogène exogènes.

2. Utilisation selon la revendication 1 caractérisée en ce que l'inhibiteur direct de la thrombine est l'argatroban.

3. Utilisation selon la revendication 1 caractérisée en ce que l'inhibiteur direct de la thrombine est la D-Phe-Pro-Arg-chlorométhylcétone.

4. Utilisation selon la revendication 1 caractérisée en ce que l'inhibiteur direct de la thrombine est le Ac-(D-Phe)Pro-boro-Arg-OH.

## Claims

1. Use of an inhibitor which acts at the active site of thrombin, for the manufacture of a medicinal product having thrombolytic activity, in the absence of exogenous plasminogen activators.

2. Use according to Claim 1, characterized in that the direct inhibitor of thrombin is argatroban.

3. Use according to Claim 1, characterized in that the direct inhibitor of thrombin is D-Phe-Pro-Arg chloromethyl ketone.

4. Use according to Claim 1, characterized in that the direct inhibitor of thrombin is Ac-(D-Phe)-Pro-boro-Arg-OH.

## Patentansprüche

1. Verwendung eines als aktive Stelle von Thrombin wirkenden Inhibitors für die Herstellung eines Arzneimittels mit thrombolytischer Wirkung in Abwesenheit von exogenen Plasminogenaktivatoren.

2. Verwendung nach Anspruch 1, dadurch gekennzeichnet, daß der direkte Thrombin-Inhibitor Argatroban ist.

3. Verwendung nach Anspruch 1, dadurch gekennzeichnet, daß der direkte Thrombin-Inhibitor D-Phe-Pro-Arg-Chlormethylketon ist.

4. Verwendung nach Anspruch 1, dadurch gekennzeichnet, daß der direkte Thrombin-Inhibitor Ac-(D-Phe)-Pro-boro-Arg-OH ist.
